# EUROPÄISCHE PATENTSCHRIFT

(11) **EP 1 151 004 B1**
(45) Veröffentlichungstag und Bekanntmachung des Hinweises auf die Patenterteilung: **07.04.2004**
(21) Anmeldenummer: 00906255.5
(22) Anmeldetag: 31.01.2000
(51) Int. Cl.: C07K 14/54, C12M 1/28, C12M 1/26

(54) **VERFAHREN ZUR HERSTELLUNG VON IL-1RA, EINEM THERAPEUTISCH WIRKSAMEN PROTEIN, AUS KÖRPERFLÜSSIGKEITEN**
METHOD FOR PRODUCING IL-1RA, A THERAPEUTICALLY ACTIVE PROTEIN FROM BODY FLUIDS
PROCEDE DE PRODUCTION D'ANTAGONISTES DES RECEPTEURS DE L'INTERLEUKINE 1, D'UNE PROTEINE A ACTIVITE THERAPEUTIQUE, A PARTIR DE FLUIDES CORPORELS

(30) Priorität: 01.02.1999 DE 19903876
(43) Veröffentlichungstag der Anmeldung: 07.11.2001
(73) Patentinhaber: ORTHOGEN Aktiengesellschaft, 40210 Düsseldorf (DE)
(72) Erfinder: REINECKE, Julio, D-50739 Köln (DE); MEIJER, Hans, D-50823 Köln (DE); WEHLING, Peter, D-40597 Düsseldorf (DE)
(74) Vertreter: Schrell, Andreas, Dr.
(86) Internationale Anmeldenummer: PCT/EP2000/000762
(87) Internationale Veröffentlichungsnummer: WO 2000/046249

(56) Entgegenhaltungen:
- EP-A- 0 088 971
- WO-A-96/01890
- WO-A-99/09051
- V RUIZ DE SOUZA ET AL.: "Selective induction of interleukin-1 receptor antagonist and interlukin-8 in human monocytes by normal polyspecific IgG (intravenous immunoglobulin)" EUROPEAN JOURNAL OF IMMUNOLOGY, Bd. 25, Nr. 5, 1995, Seiten 1267-1273, XP002088660 WEINHEIM, DE ISSN: 0014-2980
- W P AREND & D Y M LEUNG : "IgG induction of IL-1 receptor antagonist production by human monocytes" IMMUNOLOGICL REVIEWS, Bd. 139, 1994, Seiten 71-78, XP002088659 copenhagen, DK in der Anmeldung erwähnt
- CHEMICAL ABSTRACTS, vol. 125, no. 25, 16. Dezember 1996 (1996-12-16) Columbus, Ohio, US; abstract no. 325879, J PETERSEN ET AL.: "IgG for intravenous use, autologous serum and plasma induce comparable interleukin-1 receptor antagonist liberation from human mononuclear cells: an in vitro phenomenon dependin upon plastic adherence" Seite 1127; XP002086661 & AUTOIMMUNITY, Bd. 22, Nr. 2, 1995, Seiten 127-133, in der Anmeldung erwähnt
- CHEMICAL ABSTRACTS, vol. 130, no. 4, 25. Januar 1999 (1999-01-25) Columbus, Ohio, US; abstract no. 37151, H KOCH ET AL.: "Spontaneous secretion of interleukin 1 receptor antagonist (IL-1Ra) by cells isolated from herniated lumbar tissue after discectomy " XP002142041 & CYTOKINE, Bd. 10, Nr. 9, 1998, Seiten 703-705,

## Beschreibung

Die vorliegende Erfindung betrifft ein Verfahren zur Herstellung von prophylaktisch oder therapeutisch wirksamen Proteinen sowie die dabei eingesetzten Mittel, insbesondere Spritzen.

Therapeutisch wirksame Proteine wie Erythropoietin, Insulin oder Interferone, sind seit langem bekannt. Viele dieser Proteine sind bereits als Arzneimittel zugelassen und werden dementsprechend häufig eingesetzt. Aufgrund der hohen mit der Entwicklung und Zulassung dieser Medikamente verbundenen Kosten besteht jedoch ein Bedarf an einfachen und kostengünstigen Alternativen zur Bereitstellung von therapeutisch wirksamen Proteinen. Zudem sind nicht alle therapeutisch wirksamen Proteine als Arzneimittel zugelassen. Gleichwohl besteht jedoch häufig der Bedarf, auch diese Proteine dem Patienten zu applizieren. Von besonderer Bedeutung sind dabei aufgrund ihrer mutmaßlichen guten Körperverträglichkeit autologe, das heißt körpereigene, Proteine. Zu diesen Proteinen gehören der Interleukin1 Rezeptor-Antagonist, Interleukin 4, Interleukin 10 und der Tumor-Nekrose-Faktor-Rezeptor Typ I oder Typ II. Überdies weisen körpereigene Proteine den Vorteil auf, dass die natürlichen posttranslationellen Modifizierungen, wie Glycosylierungen, bereits vorhanden sind. Dies ist bei den meisten üblicherweise erhältlichen rekombinanten Proteinen nicht der Fall, da diese in prokaryotischen Wirten erzeugt werden.

Die Stimulation von Monocyten durch adhärentes Immunglobulin G zur Bildung des Interleukin-1-Rezeptor-Antagonisten wird von Arend und Leung in Immunological Reviews (1994) 139, 71-78 und Moore et al. in Am. J. Respir. Cell Mol. Biol. (1992) 6, 569-575, beschrieben. Andersen et al. in Autommunity (1995) 22, 127-133 erläutert, dass der therapeutische in vivo zu beobachtende Effekt von Immunglobulin G nicht auf eine verstärkte Bildung von Interleukin-1-Rezeptor-Antagonist zurückgeführt werden kann und dass die in vitro Bildung des Interleukin-1-Rezeptor-Antagonisten (IRAP, IL-1Ra) durch Monocyten in Abhängigkeit von an Polypropylen adsorbierten Serums- und Plasma-Bestandteilen stattfindet. Der therapeutische Einsatz von adsorbierten Serums- und Plasma-Bestandteilen zur Stimulation der Bildung therapeutisch interessanter Proteine in Therapien ist nicht nur sehr kostenlastig, sondern beinhaltet auch die Gefahr einer Kontamination mittels infektiöser Partikel, mit denen die Serumsund Plasma-Bestandteile verunreinigt sein können (Siehe auch H. Koch et al. Cytokine 10(9): 703-705 (1998) zitiert in Chem. Abs. 130(4): 37151 (1999)). Verfahren zur Herstellung unmittelbar in einer Therapie einsetzbaren IL-1Ra ohne Verwendung von adsorbierten Serums- und Plasma-Bestandteilen werden in den vorstehend erwähnten Druckschriften nicht beschrieben.

Das der vorliegenden Erfindung zugrundeliegende technische Problem besteht also darin, Verfahren und Mittel zur Herstellung von IL-1Ra bereitzustellen, die als sichere, kostengünstige und schnell durchzuführende Alternative zum Einsatz und zur Herstellung konventioneller Arzneimittelpräparate dienen.

Die Erfindung löst dieses Problem, indem ein Verfahren zur Herstellung von IL-1Ra in einer Spritze aus Glas, Quarz oder einem Kunststoff bereitgestellt wird, wobei die Spritze mit einer Körperflüssigkeit eines Organismus, zum Beispiel eines menschlichen oder tierischen Körpers, gefüllt, inkubiert und das IL-1Ra gebildet wird.

In einer bevorzugten Ausführungsform der vorliegenden Erfindung ist vorgesehen, dass die innere Struktur der Spritze aus einem besonderen Material besteht, insbesondere Glas, Kunststoff, Quarz und/oder Korund, dessen Oberfläche in einer besonders bevorzugten Ausführungsform der Erfindung modifiziert ist, insbesondere mit Hilfe eines ätzenden Agens, zum Beispiel einer Säure oder einer Lauge, insbesondere Chromschwefelsäure, und anschliessend nach Entfernen des Agens und Waschen der Spritze gegebenenfalls, das heißt in besonders bevorzugter Weise, die Oberfläche der inneren Struktur der Spritze sterilisiert wird, insbesondere durch Autoklavierung. Anschliessend wird die Spritze mit einer Körperflüssigkeit eines Patienten gefüllt, inkubiert und dabei IL-1Ra gebildet. Die mit dem Protein angereicherte Körperflüssigkeit kann dann dem Patienten wieder injiziert werden, zum Beispiel in ein krankes Gelenk. Vorzugsweise wird die Körperflüssigkeit jedoch zur Erhöhung der Reinheit des gebildeten IL-1Ra zentrifugiert und der Überstand sterilfiltriert, aliquotiert und für eine spätere Behandlung aufbewahrt. Die Erfindung sieht also in bevorzugter Ausführungsform in einem ersten Verfahrensschritt vor, dass die Oberfläche der inneren Struktur der Spritze modifiziert wird, insbesondere mit Hilfe eines ätzenden Agens, wie einer Säure oder einer Lauge, insbesondere Chromschwefelsäure, und das dann, falls erwünscht, die Oberfläche der inneren Strukturen der Spritze sterilisiert wird, insbesondere durch Autoklavierung. Vor und/oder nach der Sterilisierung kann eine Trocknung vorgesehen sein. Nach der Modifizierung und der gegebenenfalls erfolgenden Sterilisation wird die Spritze in einem zweiten Verfahrensschritt mit einer Körperflüssigkeit, insbesondere Blut, Lymphflüssigkeit, Speichel oder Urin, gefüllt und inkubiert. Vorzugsweise wird die Körperflüssigkeit dem Patienten direkt mit der Spritze entnommen. Die, vorzugsweise modifizierte, Oberfläche der inneren Struktur der Spritze induziert in der Körperflüssigkeit spezifisch, je nach eingesetztem Material, der inneren Struktur der Spritze, eingesetzter Modifizierung, insbesondere Ätzen der inneren Struktur, eingesetzter Sterilisation, insbesondere Autoklavierung, und eingesetzter Körperflüssigkeit, in quantitativ unterschiedlichem Maß die Bildung von IL-1Ra, das demgemäß in der in der Spritze vorhandenen Körperflüssigkeit angereichert beziehungsweise gebildet wird. Die so angereicherte Körperflüssigkeit kann in der Spritze steril gelagert und bei Bedarf dem Patienten direkt ohne weitere Behandlung oder vorzugsweise nach Zentrifugation und/oder Sterilfiltration wieder zugeführt werden.

Die Erfindung betrifft also auch ein Verfahren zur prophylaktischen oder therapeutischen Behandlung des menschlichen oder tierischen Körpers, zum Beispiel zur Behandlung von Rheuma, Arthrose und/oder Rückenbeschwerden, wobei dem menschlichen oder tierischen Körper eine Körperflüssigkeit, zum Beispiel Blut, mittels einer Spritze gemäß der vorliegenden Erfindung entnommen, diese Körperflüssigkeit in der Spritze inkubiert und dabei IL-1Ra gebildet oder angereichert und mittels dieser Spritze die Körperflüssigkeit wieder demselben oder einem anderen menschlichen oder tierischen Körper zugeführt wird.

Das gebildete IL-1Ra kann in vorteilhafter Weise modifiziert, zum Beispiel glycosyliert sein. Selbstverständlich erfasst die Erfindung auch die Bildung von sonstigen Modifikationen oder Varianten von IL-1Ra, wie verkürzten Formen, Mutanten oder sonstigen Derivaten.

Im Zusammenhang mit der vorliegenden Erfindung wird unter einer inneren Struktur einer Spritze jeglicher Bereich oder jegliche Struktur der Spritze verstanden, der in ihrem Inneren, das heißt im Probeaufnahmebereich, liegt und mit der aufzunehmenden Körperflüssigkeit in Kontakt kommen kann. In besonders vorteilhafter Weise ist die innere Struktur einer Spritze deren innere Oberfläche, bevorzugt eine Oberfläche mit einer Strukturierung zur Oberflächenvergrößerung. Selbstverständlich kann die vorliegende Erfindung auch mittels einer handelsüblichen Spritze ohne besondere Ausgestaltung in ihrem inneren Hohlraum durchgeführt werden. Die innere Struktur ist in einem solchen Fall die Innenfläche des Spritzenzylinders und der in dem Zylinder liegende Teil des Kolbens. Die innere Struktur kann in besonders bevorzugter Ausführungsform zusätzlich durch in den Innenraum der Spritze eingebrachte Gegenstände wie Partikel, Kugeln, Perlen, Gele, Glaswolle, Korund, Quarz, Sand, Kunststoffoder Glas-Granulat, beziehungsweise -Mehl oder ähnlichem gebildet sein, um die innere Oberfläche der Spritze zu vergrößern und so eine größere induzierende Oberfläche zur Verfügung zu stellen. Das Material, aus dem die innere Struktur besteht oder das in der inneren Struktur enthalten ist, kann ein anderes Material sein, als das, aus dem der Rest der Spritze besteht. Beispielsweise kann die Spritze aus Kunststoff bestehen und Teile ihrer inneren Struktur können zum Beispiel aus Glasgranulat sein.

Derartige zusätzliche Strukturen, wie zum Beispiel Glasperlen mit einem Durchmesser von 1 bis 5 mm, sollten gemäß einer bevorzugten Ausführungsform der Erfindung jedoch nicht mehr als 50 % des Innenvolumens der eingesetzten Spritzen einnehmen. Die eingesetzten Spritzen können beispielsweise 10 bis 100 ml Spritzen sein.

Im Zusammenhang mit der vorliegenden Erfindung wird unter einer modifizierten Oberfläche einer inneren Struktur der Spritze eine mittels eines ätzenden Agens behandelte Oberfläche verstanden, die in der Lage ist, die Bildung von IL-1Ra in einer Körperflüssigkeit eines Menschen oder Tieres zu induzieren und/oder zu verstärken. Die modifizierte Oberfläche kann sich durch eine besonders hohe Reinheit, das heißt weitgehende oder vollständige Abwesenheit von Verunreinigungen und/oder eine chemisch/physikalische Änderung der Oberflächeneigenschaften und/oder -struktur auszeichnen. Vorzugsweise wird zur Herstellung der modifizierten Oberfläche der inneren Struktur eine Lauge oder eine Säure, zum Beispiel Chromschwefelsäure, insbesondere 20 bis 80 %ige, besonders bevorzugt 50 %ige Chromschwefelsäure, eingesetzt. Die Spritze wird in bevorzugter Weise 5 bis 30 min mit dem ätzenden Agens, insbesondere der Chromschwefelsäure, inkubiert.

Nach Entfernen des Agens können bevorzugt ein oder mehrere Waschschritte erfolgen sowie gegebenenfalls vorzugsweise eine Sterilisation, beispielsweise durch Autoklavierung, insbesondere Autoklavierung bei 100°C bis 150°C für 20 bis 60 min unter einem Druck von 1 bis 5 bar, durchgeführt werden. Nach dem Waschen und vor und/oder nach dem Autoklavieren können gegebenenfalls noch ein oder mehrere Trocknungsschritte bei 60 bis 150°C, vorzugsweise 80°C, für 30 bis 120 min in zum Beispiel einem Trockenschrank erfolgen.

In besonders vorteilhafter Ausgestaltung der Erfindung ist vorgesehen, dass die Spritze, insbesondere die innere Struktur der Spritze, aus Glas, zum Beispiel Quarzglas, Korund, Quarz, einem Kunststoff, wie Polystyrol, Polyethylen, Polyvinylchlorid, Polypropylen, oder einem ähnlichen Stoff hergestellt ist, das heißt aus diesen Stoffen besteht oder diese Stoffe oder Gemische davon im wesentlichen enthält.

Überraschenderweise konnte im Rahmen der vorliegenden Lehre gezeigt werden, dass eine Spritze aus Glas und/oder eine innere Struktur aus Glas, insbesondere Glasgranulat, eine besonders stark induzierende Wirkung aufweist.

In einer besonders bevorzugten Ausführungsform wird vor der Behandlung, das heißt vor der Abnahme der Körperflüssigkeit, erhitztes Glas verwendet, insbesondere auf 100°C-210°C, insbesondere 170° - 200°C, vorzugsweise 180°C erhitztes Glas, das selbstverständlich vor der erfindungsgemäßen Verwendung abgekühlt wird. In besonders bevorzugter Ausführungsform kann das Glas in Form von Glasmehl oder Glasgranulat vorliegen.

Die inneren Strukturen können aber auch aus Quarz, zum Beispiel Quarzmehl oder Quarzsand und/oder Korund, zum Beispiel in Form einer Suspension in Wasser, bestehen oder diese enthalten.

In besonders bevorzugter Ausführungsform weisen diese Stoffe nach gegebenenfalls erfolgter Modifizierung, insbesondere Ätzung, und nach der gegebenenfalls erfolgenden Sterilisation, IL-1Ra-induzierende Eigenschaften auf.

In einer weiteren bevorzugten Ausführungsform der Erfindung ist vorgesehen, die innere Struktur einer Spritze mit einer modifizierbaren und sterilisierbaren Beschichtung zu versehen und anschliessend die Modifizierung beziehungsweise auch gegebenenfalls die Sterilisierung vorzunehmen.

Die vorliegende Erfindung ist vorteilhaft insofern, als dass ein einfach durchzuführendes Verfahren bereitgestellt wird, mit dem autologes, durch Induktion, insbesondere Oberflächen-Induktion, herstellbares IL-1Ra hergestellt werden kann und in der so hergestellten Form, das heißt zusammen mit den anderen Bestandteilen der in der Spritze befindlichen Flüssigkeit dem Patienten direkt, das heißt ohne weitere Manipulation, wie zum Beispiel Umfüllen in andere Behälter, wieder appliziert werden können. In bevorzugter Ausführungsform kann zur Abtrennung von festen Bestandteilen eine Zentrifugation und/oder Sterilfiltration vorgesehen werden, bevor das gebildete IL-1Ra appliziert wird. Der Einsatz kommerziell erhältlicher oftmals teuerer Arzneimittel wird daher überflüssig. Schließlich erweist sich die Erfindung als vorteilhaft, als dass eine Kontamination, Verunreinigung, Infektion oder ähnliches des IL-1Ra vermieden wird, die auf einer außerhalb des Patienten stattfindenden Arzneimittelherstellung beruht.

Die vorliegende Erfindung sieht in einer besonders bevorzugten Ausführungsform also ein Verfahren zur Herstellung des Interleukin-1 Rezeptor-Antagonisten vor, wobei die inneren Strukturen der Spritze aus einem besonders behandelten Material bestehen, insbesondere Glas, Quarz oder Kunststoff, wobei die Oberfläche der inneren Strukturen der Spritze modifiziert wurde, insbesondere mit Hilfe eines ätzenden Agens, insbesondere Chromschwefelsäure, wobei gegebenenfalls anschliessend die Oberfläche der inneren Strukturen der Spritze sterilisiert wurde, insbesondere durch Autoklavierung, und wobei die Spritze mit einer Körperflüssigkeit, vorzugsweise Blut, gefüllt, inkubiert und in der Körperflüssigkeit IL-1Ra gebildet und angereichert wird. Unter anderem aufgrund der besonderen Modifizierung der Oberfläche der inneren Strukturen der Spritze, insbesondere Ätzung, und der besonderen Sterilisation der Oberfläche, insbesondere Autoklavierung, ist die erfindungsgemäße Spritze in der Lage, die im Blut vorhandenen Monocyten zur Bildung des IL-1Ra anzuregen, so dass dieses im Blut angereichert wird. Das sich in der Spritze befindende Blut kann in einer Ausführungsform der vorliegenden Erfindung nach Inkubation, das heißt nach Anreicherung des IL-1Ra, direkt, ohne weitere Manipulation, wie zum Beispiel Umfüllen, wieder dem Patienten zugeführt werden, dem das in die Spritze eingefüllte Blut entnommen worden war.

Vorteilhafterweise kann in besonders bevorzugter Ausführungsform der vorliegenden Erfindung zur Abtrennung fester Bestandteile, wie Zellen, eine Zentrifugation und/oder Sterilfiltration des sich in der Spritze befindenden Bluts vorgesehen werden. Anschließend wird gegebenenfalls aliquotiert. Die Erfindung sieht also auch vor, dass dem Patienten das Blut mittels der mit einer besonders modifizierten und sterilisierten, insbesondere autoklavierten Oberfläche der inneren Strukturen ausgestatteten Spritze entnommen, das Blut in der Spritze inkubiert und nach IL-1Ra-Bildung demselben oder einem anderen Patienten wieder mit der Spritze zugeführt werden kann. Eine derartige Vorgehensweise ist zum Beispiel besonders vorteilhaft im Bereich der Neuroorthopädie, das heißt beispielsweise bei neurologisch bedingten Rückenbeschwerden. Für die Behandlung derartiger Beschwerden kam bisher nur eine Bandscheibenoperation, Cortisonbehandlungen, Spülungen mit Kochsalzlösungen oder ähnliches in Betracht. Die erfindungsgemäße Bereitstellung von IL-1Ra, das heißt insbesondere autologem IL-1Ra, ermöglicht auch die besonders einfache, kostengünstige und effektive Behandlung von Rheuma und Arthrose.

Die Erfindung sieht in einer weiteren Ausführungsform vor, dass die innere Struktur der Spritze zusätzlich mit Anticoagulantien beschichtet wird, insbesondere Heparin, Citrat, EDTA, CPDM oder CPDA. Überraschenderweise konnte im Rahmen der vorliegenden Lehre gezeigt werden, das bei Verwendung von Heparin als Anticoagulans eine gute Induktion erzielt wird. Erfindungsgemäß kann auch vorgesehen sein, die Anticoagulantien nicht als Beschichtung, sondern ungebunden im Behälter einzusetzen, zum Beispiel in lyophilisiertem oder flüssigem Zustand in die Spritze zu geben.

In einer besonders bevorzugten Ausführungsform der vorliegenden Erfindung wird in der Spritze jedoch kein Anticoagulans, insbesondere kein Heparin, eingesetzt. Dies führt zu einer nochmals verbesserten Inkubation.

Die Erfindung sieht in einer weiteren bevorzugten Ausführungsform vor, die Inkubation der Körperflüssigkeit in der Spritze über einen Zeitraum von 12 bis 72 Stunden, bevorzugt 24 Stunden, vorzugsweise bei Raumtemperatur, also 20°C bis 41°C, insbesondere bei 37°C, durchzuführen.

Die Erfindung sieht in einer Ausgestaltung der Erfindung auch vor, dass nach Bildung des therapeutisch oder prophylaktisch wirksamen Proteins in der Körperflüssigkeit die Körperflüssigkeit weiter behandelt wird, um beispielsweise bestimmte Bestandteile dieser abzutrennen, zum Beispiel Blutplasma oder Blutplättchen. Diese Abtrennung kann in bevorzugter Ausführungsform der Erfindung durch Zentrifugation oder Filtration durchgeführt werden.

Die Erfindung betrifft in einer weiteren Ausführungsform ein Verfahren zur Herstellung einer zur In-vitro-Induktion von prophylaktisch oder therapeutisch wirksamen Proteinen, insbesondere des Interleukin-1 Rezeptor-Antagonisten, geeigneten Spritze, wobei sich die Spritze durch das besonders behandelte Material der inneren Struktur der Spritze, insbesondere Kunststoff oder Glas, auszeichnet. Insbesondere ist erfindungsgemäß vorgesehen, die Oberfläche der inneren Struktur der Spritze mittels eines ätzenden Agens, insbesondere einer Lauge oder einer Säure zu ätzen, insbesondere mit Hilfe von Chromschwefelsäure. Nach Entfernen des ätzenden Agens und Waschen der Spritze kann vorgesehen sein, die Oberfläche der inneren Struktur zu sterilisieren, insbesondere zu autoklavieren. Vorzugsweise kann auch vorgesehen sein, eine, vorzugsweise aus Glas hergestellte, Spritze vor ihrer Verwendung zu erhitzen, zum Beispiel auf 100°C bis 210°C, besonders 170°-200°C.

Die Erfindung betrifft selbstverständlich auch die so hergestellte Spritze, die in besonders bevorzugter Ausführungsform aus Glas, Quarz oder Kunststoff, insbesondere Polystyrol, Polyvinylchlorid, Polyethylen oder Polypropylen, hergestellt ist, wobei sich die Spritze durch die besondere Be-Handlung der Oberfläche der inneren Struktur der Spritze, insbesondere hergestellt aus Glas, Quarz oder Kunststoff, auszeichnet, die mittels Einwirkung eines ätzenden Agens ausgeführt wird. In bevorzugter Weise wurde die Spritze vor Ihrer Verwendung erhitzt, vorzugsweise auf 100°C-210°C, zum Beispiel 170°C-200°C.

Die Erfindung betrifft auch die Verwendung von Lauge oder Säure, insbesondere Chromschwefelsäure, zum Modifizieren der Oberfläche der inneren Strukturen der erfindungsgemäßen Spritzen, vorzugsweise aus Polystyrol, Polyvinylchlorid, Polyethylen, Polypropylen, Quarz oder Glas, zur In-vitro-Induktion von prophylaktisch oder therapeutisch wirksamen Proteinen, vorzugsweise dem Interleukin-1 Rezeptor-Antagonisten.

Die Erfindung betrifft auch die Verwendung von oberflächenvergrößernden Vorrichtungen beziehungsweise Substanzen wie Glasmehl, Glasgranulat, Quarzmehl, Quarzsand, Korund, Kügelchen, Perlen, Sand etc. zur Verwendung in einem vorgenannten Verfahren, das heißt insbesondere zur Verwendung als Induktor für die IL-1Ra-Bildung in einem Gefäß, zum Beispiel einer Spritze.

Weitere vorteilhafte Ausgestaltungen der Erfindung ergeben sich aus den Unteransprüchen.

Die Erfindung wird anhand von Figuren und Ausführungsbeispielen näher erläutert.

Die Figuren zeigen:
Die Figur 1 zeigt in schematischer Darstellung eine erfindungsgemäße Spritze.
Die Figuren 2 bis 12 zeigen die IL-1Ra Bildung in einer erfindungsgemäß eingesetzten Spritze bei verschiedenen Bedingungen.
Die Fig. 13 und 14 zeigen, dass durch Waschen der Spritze das eingesetzte ätzende Agens vollständig entfernt wird.

### Beispiel 1: Herstellung und Verwendung einer Glasspritze mit Granulat

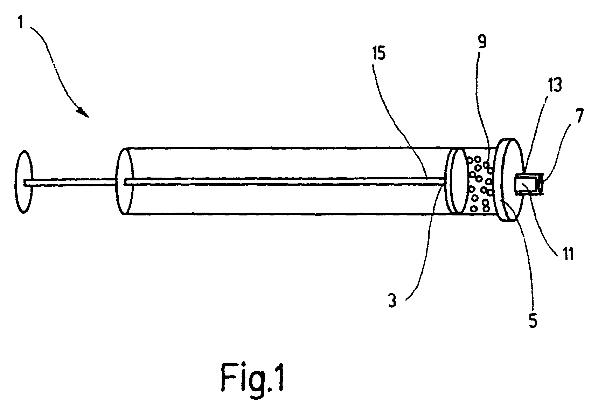

Die Figur 1 zeigt eine 50 ml Spritze 1 aus Glas (Fortuna Optima, Best.Nr. 7.102.-44, wenn im Folgenden nichts anderes angegeben, wurde diese Spritze in allen Beispielen eingesetzt) mit einem Kolben oder Stempel 3, einem abschraubbaren Verschluß 5 mit einem Verschlußansatz 13 (male Luer) und einer auf dem Verschlußansatz 13 angeordneten und diesen abschließenden abnehmbaren Kappe 7 mit Septum. Der Stempel 3 weist eine Sollbruchstelle 15 auf. So ist es möglich, nach Abbrechen des Stempels die Spritze direkt zu zentrifugieren. Dargestellt ist auch Granulat 9 aus Glas (Glasperlen der Firma Roth, Art.Nr. A 557.1). Die Größe der Granulatpartikel 9 liegt zwischen 1 und 3 mm Durchmesser, wobei jedoch auch kleinere Partikel, insbesondere größer als 100 µm, eingesetzt werden können, z.B. Glasmehl. Selbstverständlich können auch Spritzen, z.B. aus Glas oder Kunststoff, eingesetzt werden, die keine Sollbruchstelle im Stempel aufweisen.

Zur Herstellung der Spritze 1 wird die Oberfläche der inneren Struktur der fabrikneuen und originalverpackten Spritze 1 und des fabrikneuen und originalverpackten Granulats 9 modifiziert mit Hilfe eines handelsüblichen Chromschwefelsäure-Präparat, indem das Chromschwefelsäure-Präparat in die Spritze aufgenommen wird und die Spritzeninnenwand, das heißt Zylinderinnenwand und Kolben, sowie das Granulat damit geätzt werden. Die Spritze wird durch ein- bis zehnmaliges, vorzugsweise dreimaliges, vollständiges Aufziehen und Ausspritzen von 50 %iger Chromschwefelsäure (Merck, Darmstadt, Best.Nr. 1.02499.2500, Chromschwefelsäure wird mit Biochrom Reinstwasser Ultra Pure Water No. L 0040 bis zur gewünschten Verdünnung verdünnt) behandelt und dabei gesäubert beziehungsweise modifiziert. Nach dem letzten Aufziehen wird die Spritze unten abgedichtet und in gefülltem Zustand 5 bis 30 min mit der Chromschwefelsäure inkubiert. Anschliessend wird der Spritzenkolben entfernt und zwei- bis zehnmal, vorzugsweise viermal, durch vollständiges Auffüllen und Ablaufenlassen des Spritzenzylinders mit frischem Reinstwasser durchgewaschen, wobei darauf zu achten ist, dass das Waschwasser vollständig ein- und ausgefüllt wird. Sodann wird der Spritzenkolben in 50 %ige Chromschwefelsäure getaucht und gründlich mit destilliertem Wasser abgewaschen.

Eventuell in der Spritze vorhandene Wasserreste werden durch Betupfen des Lueranschlusses kapillar abgesaugt, um ein schnelles Trocknen der Spritze zu gewährleisten. Voneinander getrennte Kolben und Spritzen inklusive der eventuell darin enthaltenen Glasperlen werden in Melag-Folie mit Indikatorfeld (Melag, Melafol 1502) eingeschweißt (Melag, Melaseal). Die so verpackten Spritzen werden im Trockenschrank (Melag-Trockensterilisator) bei 80°C für mindestens 60 min getrocknet. Die getrockneten verpackten Spritzen werden anschliessend bei 132°C 30 min bei 2 bar autoklaviert (Wolf Autoclav HRM 242 II) und bei 80°C für mindestens 60 min ein weiteres Mal getrocknet.

Vor der Blutentnahme (siehe unten) wird Heparin (Liquemin N 2500, Heparin-Natrium 2500 I.E.) oder Citrat (ACDA) in die Spritze eingebracht, um eine Koagulation des später aufgenommenen Blutes zu verhindern. Der Einsatz von Coagulantien kann sich als vorteilhaft bei der Aufarbeitung von IL-1Ra-haltigem Serum erweisen.

Die Spritze 1 wird eingesetzt, indem Blut eines Patienten mit Hilfe eines nicht dargestellten Adapters entnommen wird, der die abschraubbare Kappe 7 mittels eines nicht dargestellten Schlauches mit einer nicht dargestellten Kanüle verbindet. Der Adapter weist eine Nadel auf, mittels derer das im Verschlußansatz 13 vorhandene Septum durchstochen wird. Anschliessend wird der Adapter abgenommen und eine Inkubation des Vollblutes bei 37°C für 24 Stunden unter dem Schutz der abnehmbaren Kappe 7, deren Septum sich selbsttätig geschlossen hat, durchgeführt. Die Inkubation kann stehend oder liegend erfolgen. Erfolgt die Inkubation stehend, wird das Plasma durch das Septum und einen sterilen Vorsatzfilter (0,2 µm) abgenommen. Zusätzlich oder alternativ kann eine Zentrifugation vorgesehen werden. Erfolgt die Inkubation liegend, wird das Blut zentrifugiert und das Plasma durch einen sterilen Vorsatzfilter (0,2 µm) abgenommen. Es kann aber auch vorgesehen sein, das Plasma durch das Septum abzunehmen ohne eine Zentrifugation durchzuführen. Anschliessend wird das Plasma zum Beispiel an einer Nervenwurzel oder einem Gelenk des Patienten reinjiziert.

### Beispiel 2: Herstellung und Verwendung einer Kunststoff-Spritze mit Granulat

In diesem Beispiel wird steriles Granulat aus Polystyrol, Glas oder einem anderen modifizierbaren und/oder sterilisierbaren Material eingesetzt. Die Oberfläche des Granulats wird mit Hilfe eines handelsüblichen Chromschwefelsäure-Präparat im Batch-Verfahren, wie im Beispiel 1 ausgeführt, modifiziert. Anschliessend wird das Granulat mit Wasser gespült, um die Chromschwefelsäure-Reste wegzuwaschen. Dann wird das Granulat bei 121°C unter einem Druck von 2 bar mindestens 20 min inkubiert, um so das Granulat zu sterilisieren und mit Wasser zu sättigen. Das Granulat wird anschliessend getrocknet bei 80°C für 20 min.

Eine herkömmliche, nicht modifizierte, fabrikneue und originalverpackte Polypropylenspritze (50 ml, Becton Dickinson, Heidelberg, Art. Nr. 00137) wird mit dem modifizierten und sterilisierten Granulat (1, 2, 4 oder 10 cm³) sowie mit einer hinreichenden Menge eines Antikoagulans wie Heparin (Liquemin, Heparin-Natrium 2500 I.E.) oder Citrat (zum Beispiel ACDA) befüllt.

Die befüllte Spritze wird inklusive Abnahmekanüle und Schlauch verpackt und anschliessend Gamma- oder Elektronen-sterilisiert.

Der Anwender entnimmt das sterile Besteck und entnimmt dem Patienten Blut. Die Spritze verfügt an ihrer Öffnung in dem Verschlußansatz über ein Septum, welches zur Entnahme durch das Abnahmezubehör, also die Nadel des Adapters, durchstochen wird. Nach Abnahme des Adapters verschließt sich das Septum selbsttätig wieder. Nach Blutentnahme wird der Spritzenstempel an einer Sollbruchstelle abgebrochen.

Die Spritze mit Blut wird 24 Stunden bei 37°C bis 41°C inkubiert.
a) Erfolgt die Inkubation stehend, wird das Plasma durch das Septum und einen sterilen Vorsatzfilter, zum Beispiel 0,2 µm, abgenommen.
b) Erfolgt die Inkubation liegend, wird nach Zentrifugation der Spritze das Plasma durch einen sterilen Vorsatzfilter, zum Beispiel 0,2 µm, abgenommen.

Die Reinjektion des Plasma erfolgt zum Beispiel an einer Nervenwurzel, ins Gelenk oder in die Bandscheibe.

### Beispiel 3: Herstellung und Verwendung einer Spritze ohne Granulat

Eine originalverpackte, fabrikneue Spritze aus einem modifizierbaren, sterilisierbaren Material (5, 10, 20 oder 50 ml) wird, wie in Beispiel 1 angegeben, mit Chromschwefelsäure modifiziert und anschliessend autoklaviert sowie getrocknet. Vorzugsweise besteht die Spritze aus Glas, Polystyrol, oder einem speziell modifizierbaren anderen Material.

Die modifizierte und sterilisierte Spritze wird mit einer hinreichenden Menge Heparin (Liquemin, Heparin-Natrium 2500 I.E.) oder Citrat (ACDA) befüllt.

Die befüllte Spritze wird inklusive Abnahmekanüle und Schlauch anschliessend Gamma- oder Elektronen-sterilisiert.

Der Anwender entnimmt das sterile Besteck und entnimmt dem Patienten Blut. Die Spritze verfügt an der Öffnung über ein in dem Verschlußansatz enthaltendes Septum, welches zur Entnahme durch das Abnahmezubehör, also den eine Nadel aufweisende Adapter durchstochen wird. Nach Abnahme des Adapters verschließt sich das Septum selbsttätig wieder. Nach Blutentnahme wird der Spritzenstempel an einer Sollbruchstelle abgebrochen.

Die Spritze mit Blut wird 24 Stunden bei 37°C bis 41°C inkubiert.
a) Erfolgt die Inkubation stehend (zum Beispiel in einem Reagenzglasständer), wird das Plasma durch das Septum abgenommen, wobei eine Filtration durch einen sterilen Vorsatzfilter, zum Beispiel 0,2 µm, erfolgt.
b) Erfolgt die Inkubation liegend, wird nach Zentrifugation der Spritze das Plasma durch das Septum abgenommen, wobei dabei durch einen sterilen Vorsatzfilter, zum Beispiel 0,2 µm, eine Filtration erfolgt.

Die Reinjektion des Plasma erfolgt zum Beispiel an einer Nervenwurzel oder ins Gelenk oder in die Bandscheibe.

### Beispiel 4: Herstellung des Interleukin-1 Rezeptor-Antagonisten in einer Spritze unter Verwendung von Heparin

Eine kommerziell erhältliche fabrikneue und originalverpackte Spritze, bestehend aus Glas, wurde mit Chromschwefelsäure gefüllt und für 20 min bei Raumtemperatur inkubiert, wie in Beispiel 1 angegeben. Anschliessend wurde die Spritze viermal mit destilliertem Wasser gespült, verpackt, 30 min bei 131°C unter einem Druck von 2 bar autoklaviert und 30 min bei 100°C getrocknet.

Nach Abschluss der Modifizierung und Sterilisation wird die Spritze zwischengelagert. Arzneimittelrechtlich zugelassenes Heparin (Liquemin, Heparin-Natrium 2500 I.E.) wird in die Spritze als Anticoagulanz aufgezogen.

Anschliessend wird dem Patienten venöses Blut mit der beschichteten Spritze steril entnommen.

Die Spritze wird bei Raumtemperatur 12 bis 72 Stunden inkubiert. In dieser Zeit findet eine starke Anreicherung im Plasma enthaltender Proteine, insbesondere des Interleukin-1 Rezeptor-Antagonisten, im Blutplasma statt. Es konnte eine Konzentration von 1 bis 50 ng/ml des Interleukin-1 Rezeptor-Antagonisten festgestellt werden.

Anschliessend wird das Blut oder das Plasma dem Patienten mit der beschichteten Spritze injiziert.

### Beispiel 5: Herstellung des Interleukin-1 Rezeptor-Antagonisten in einer Spritze ohne Verwendung von Heparin

Eine kommerziell erhältliche fabrikneue und originalverpackte Spritze, bestehend aus Glas, wurde mit Chromschwefelsäure gefüllt und für 20 min bei Raumtemperatur inkubiert, wie in Beispiel 1 angegeben. Anschliessend wurde die Spritze viermal mit destilliertem Wasser gespült, verpackt, 30 min bei 131°C unter einem Druck von 2 bar autoklaviert und 30 min bei 100°C getrocknet.

Nach Abschluss der Modifizierung und Sterilisation wird die Spritze zwischengelagert.

Anschliessend wird dem Patienten venöses Blut mit der Spritze steril entnommen.

Die Spritze wird bei Raumtemperatur 12 bis 72 Stunden inkubiert. In dieser Zeit findet eine starke Anreicherung im Plasma enthaltender Proteine, insbesondere des Interleukin-1 Rezeptor-Antagonisten, im Blutplasma statt. Es konnte eine Konzentration von 1 bis 50 ng/ml des Interleukin-1 Rezeptor-Antagonisten festgestellt werden.

Anschliessend wird das verdünnte Blut oder der Kulturüberstand dem Patienten injiziert.

### Beispiel 6: Herstellung des IL-1Ra in einer Polystyrol-Microtiter-Platte mit Granulat

Zur Herstellung des Granulats wird das fabrikneue und originalverpackte Granulat im Batch-Verfahren modifiziert mit Hilfe eines handelsüblichen Chromschwefelsäure-Präparates, indem das Granulat in einen Behälter (wie zum Beispiel ein 250 ml Glasbecher XXX) gebracht wird, dem ein 50 %iges Chromschwefelsäure-Präparat (Merck, Darmstadt, Best. Nr. 1.02499.2500, Chromschwefelsäure wird mit Biochrom Reinstwasser Ultra Pure Water No. L 0040 bis zur gewünschten Verdünnung verdünnt) hinzugefügt wird und das Granulat behandelt und dabei gesäubert beziehungsweise modifiziert wird. Das Granulat wird 5 bis 60 Minuten mit der Chromschwefelsäure inkubiert.

Anschliessend wird die Chromschwefelsäure entfernt und die Kugeln zwei- bis zehnmal mit frischem Reinstwasser durchgewaschen. Es wird so lange gewaschen, bis in dem vorletzten Waschschritt der pHund der Leitwert des Reinstwassers und des Waschwassers gleich sind.

Anschließend wird das Granulat in einem Glasbecher (wie zum Beispiel ein 250 ml Glasbecher XXX) bei 132°C 30 Min bei 2 bar autoklaviert (Wolf Autoclav HRM 242 II) und bei 60°C bis 100°C, vorzugsweise bei 80°C, 30 Min getrocknet. Alternativ wird das Granulat direkt bei 120°C bis 210°C, vorzugsweise bei 180°C für 30 Minuten Hitze-sterilisiert (Melag-Trockensterilisator).

Für die Blutentnahme werden benutzt: Sarstedt Monovetten mit EDTA, Citrat, CPDA, CPDM oder Heparin, um eine Koagulation des später aufgenommenen Blutes zu verhindern.

3 bis 12 Glasperlen werden in je ein Näpfchen einer Mikrotiterplatte gegeben (Nunc, Art. Nr. 150 687), 1 ml Blut möglichst schnell nach der Abnahme hinzugegeben.

Nach 24 Std. Inkubation (37°C, 5 % CO₂) (Hereaus Inkubator):
Über Nacht Blutkuchen sedimentiert, 300 µl Serum werden, ohne feste Bestandteile aufzuwirbeln beziehungsweise aufzunehmen, abgenommen und die IL-1Ra Proteinkonzentration bestimmt (ELISA, R&D, Wiesbaden, Quantikine Human IL-1Ra).

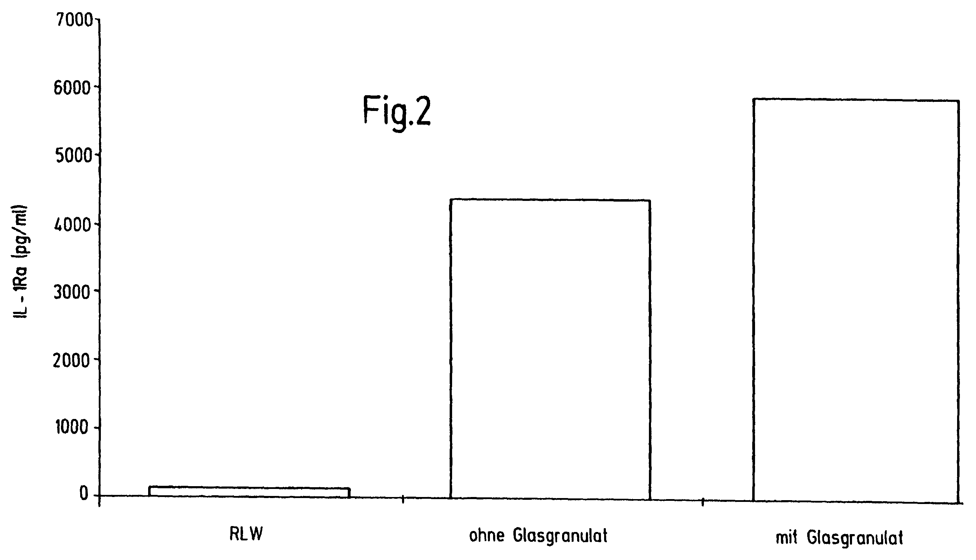

### Fig. 2: IL-1Ra Produktion in einer Glasspritze mit und ohne Glasgranulat

| Methode | |
|---|---|
| IL-1Ra Produktion | Verwendung einer Glasspritze wie beschrieben in Beispiel 1 Messungen an einem Patienten |
| | |
| Glaskugeln | Roth, Chromschwefelsäure behandelt, gewaschen und autoklaviert |
| | |
| RLW | Reaktionsleerwert, IL-1Ra Konzentration bei Abnahme (vor IL-1Ra Produktion) |

### Ergebnis

### Hinzufügung des Glasgranulats erhöht die IL-1Ra Produktion

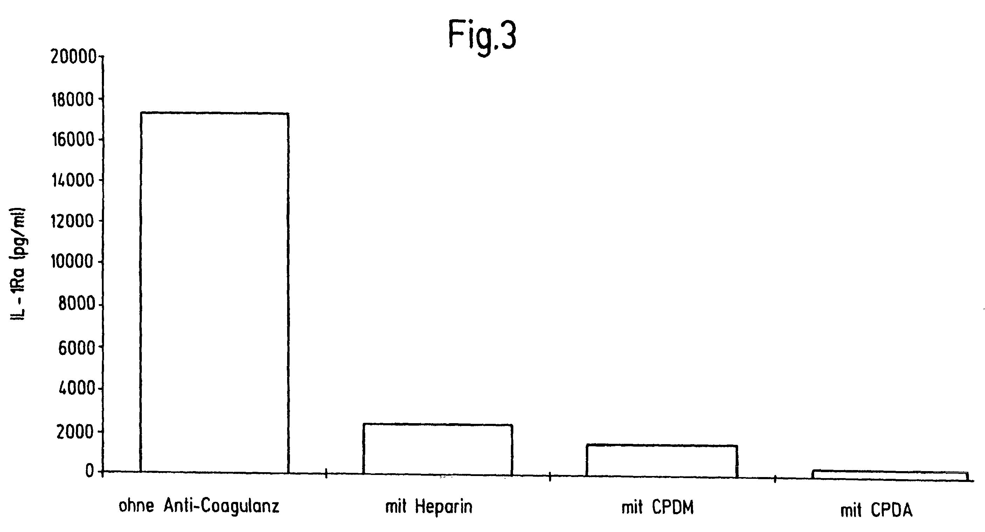

### Fig. 3 IL-1Ra Produktion in einer Glasspritze mit und ohne Anti-Coagulanz

| Methode | |
|---|---|
| IL-1Ra Produktion | Verwendung einer Glasspritze wie beschrieben in Beispiel 1 Messungen an einem Patienten |
| | |
| Glaskugeln | Roth, Chromschwefelsäure behandelt, gewaschen und autoklaviert |
| | |
| CPDM | Citrat Phosphat Dextrose Mannit |
| | |
| CPDA | Citrat Phosphat Dextrose Adenin |

### Ergebnis

Hinzufügung unterschiedlicher Anti-Coagulantia beeinflusst die Effektivität der IL-1Ra Produktion in unterschiedlichem Maße.

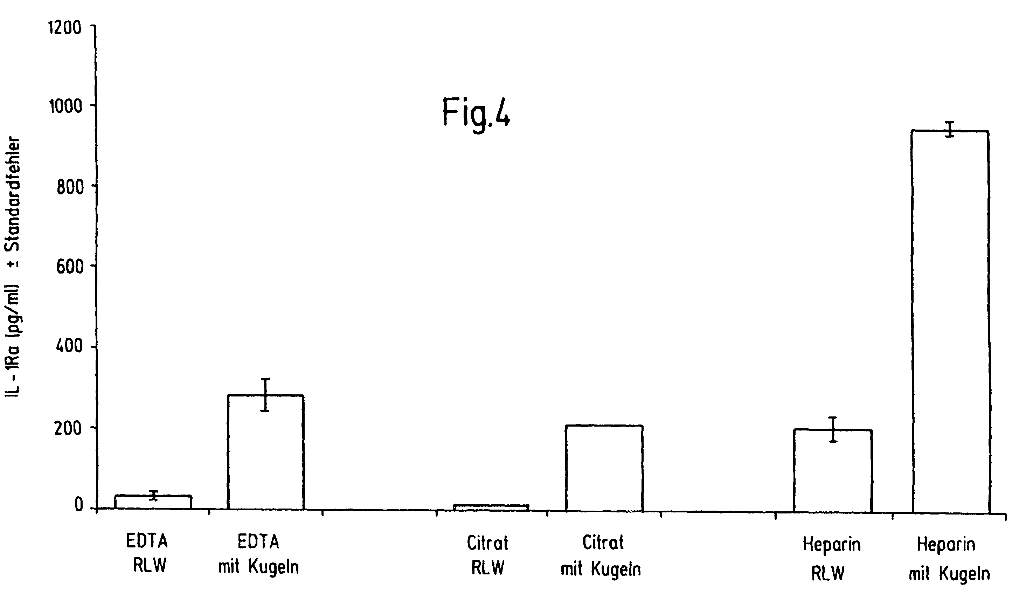

### Fig. 4 IL-1Ra Produktion in einer Mikrotiterplatte mit und ohne Anti-Coagulanz

| Methode | |
|---|---|
| IL-1Ra Produktion | Verwendung einer Mikrotiterplatte wie beschrieben in Beispiel 6 Messungen an einem Patienten |
| | |
| Glaskugeln | Duran, Chromschwefelsäure behandelt, gewaschen und autoklaviert. Es wurden 12 Kugeln 1 ml Vollblut hinzugefügt. |
| | |
| RLW . | Reaktionsleerwert, IL-1Ra Konzentration bei Abnahme (vor der IL-1Ra Produktion) |

### Ergebnis

Hinzufügung unterschiedlicher Anti-Coagulantia beeinflusst die Effektivität der IL-1Ra Produktion in unterschiedlichem Maße

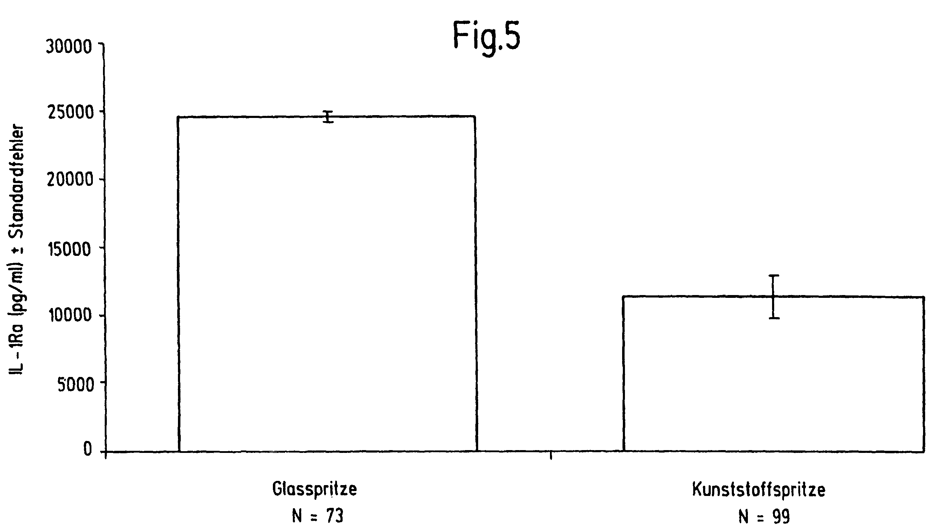

### Fig. 5. IL-1Ra Produktion in einer Glas- und Kunststoffspritze mit Glasgranulat in einem Patientenkollektiv

| Methode | |
|---|---|
| IL-1Ra Produktion | Verwendung einer Glasspritze wie beschrieben in Beispiel 1 und 2 |
| | Messungen in einem orthopädischen Patientenkollektiv |
| | |
| Glaskugeln | Roth, Chromschwefelsäure behandelt, gewaschen und autoklaviert |

### Ergebnis

Sowohl in einer Glas- als auch Kunststoffspritze kann IL-1Ra produziert werden, jedoch ist die Effektivität der IL-1Ra Produktion in einer Glasspritze signifikant höher

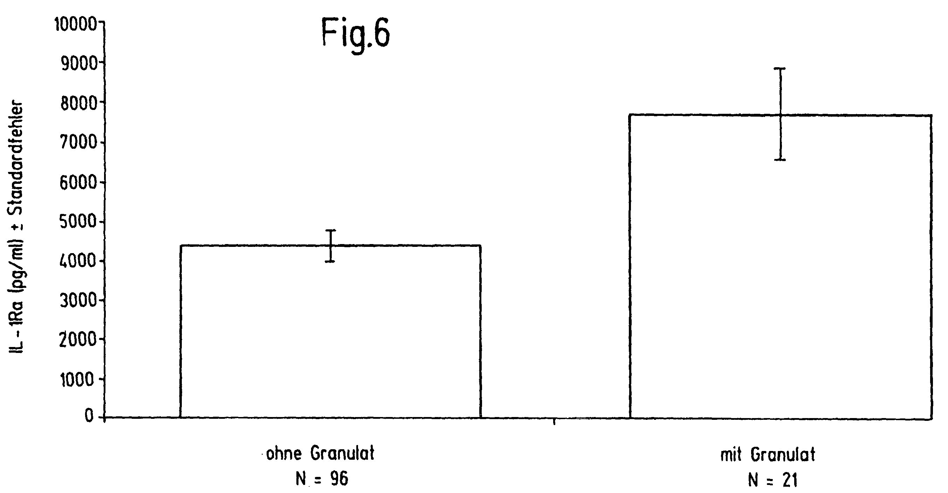

### Fig. 6 IL-1Ra Produktion in einer Glasspritze mit und ohne Glasgranulat in einem Patientenkollektiv

| Methode | |
|---|---|
| IL-1Ra Produktion | Verwendung einer Glasspritze wie beschrieben in Beispiel 1 Messungen in einem orthopädischen Patientenkollektiv |
| | |
| Glaskugeln | Roth, Chromschwefelsäure behandelt, gewaschen und autoklaviert |

### Ergebnis

Sowohl mit als auch ohne Glasgranulat kann IL-1Ra in einer Glasspritze produziert werden, jedoch ist die Effektivität der IL-1Ra Produktion mit Glasgranulat signifikant höher

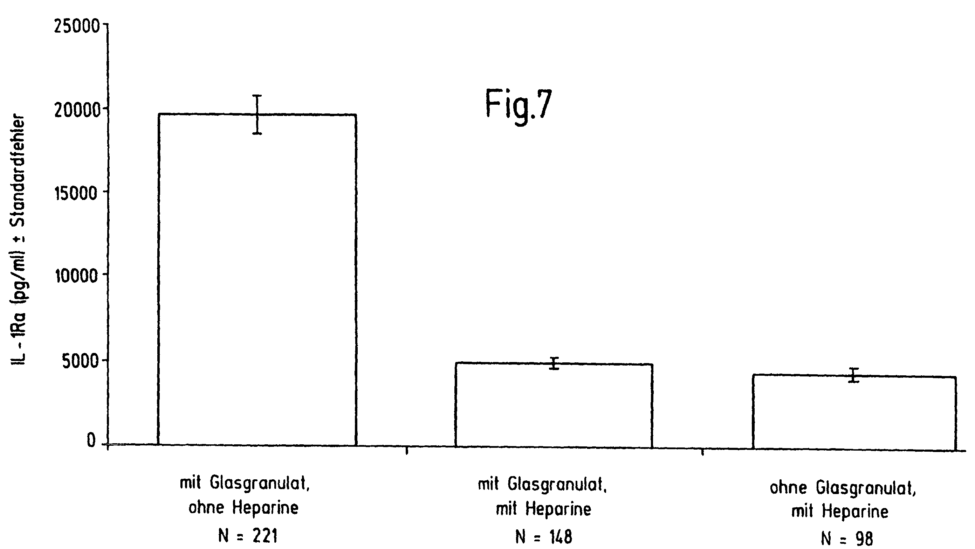

### Fig. 7 IL-1Ra Produktion in einer Glasspritze mit und ohne Glasgranulat und mit und ohne Heparin in einem Patientenkollektiv

| Methode | |
|---|---|
| IL-1Ra Produktion | Verwendung einer Glasspritze wie beschrieben in Beispiel 1 Messungen in einem orthopädischen Patientenkollektiv |
| | |
| Glaskugeln | Roth, Chromschwefelsäure behandelt, gewaschen und autoklaviert |

### Ergebnis

Sowohl mit als auch ohne Glasgranulat sowie auch mit als auch ohne Heparin kann in einer Glasspritze IL-1Ra produziert werden, jedoch ist die Effektivität der IL-1Ra Produktion mit Glasgranulat ohne Heparin signifikant höher

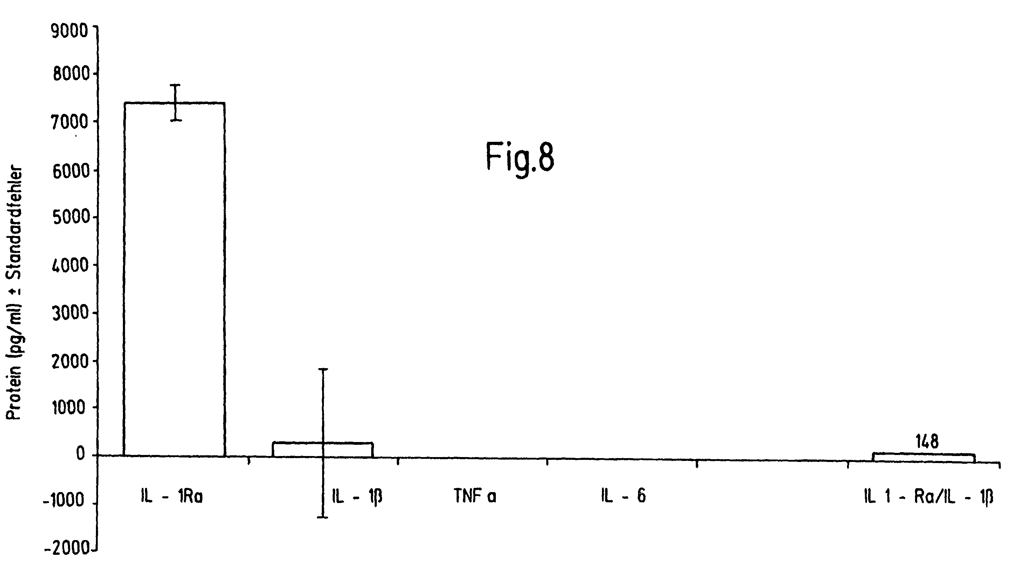

### Fig 8 Proteinproduktion unter Verwendung einer Glasspritze mit Glasgranulat in einem Patientenkollektiv

| Methode | |
|---|---|
| Proteinproduktion | Verwendung einer Glasspritze wie beschrieben in Beispiel 1 Messungen in einem orthopädischen Patientenkollektiv |
| | |
| Glaskugeln | Roth, Chromschwefelsäure behandelt, gewaschen und autoklaviert |
| | |
| TNFa | Tumor Necrosis Factor alpha |
| | |
| IL-6 | Interleukin-6 |
| | |
| ELISA | Die IL-1, TNFa und IL-6 Konzentrationen wurden mit Hilfe eines Kombo-Kits von R&D bestimmt |

### Ergebnis

Mit Hilfe der beschriebenen Methode wird spezifisch IL-1Ra produziert. TNF-alpha und IL-6 Produktion konnten nicht nachgewiesen werden. In geringen Mengen wird auch IL-1β produziert. Da das Verhältnis IL-1Ra : IL-1β, das erwartungsgemäß für eine klinisch-therapeutische Wirkung des produzierten IL-1Ra mehr als 100 sein soll (W.P. Arend et al., 1998, Annu. Rev. Immo.16m 27 - 55) im Durchschnitt bei 148 liegt, ist der autolog produzierte IL-1Ra therapeutisch wertvoll.

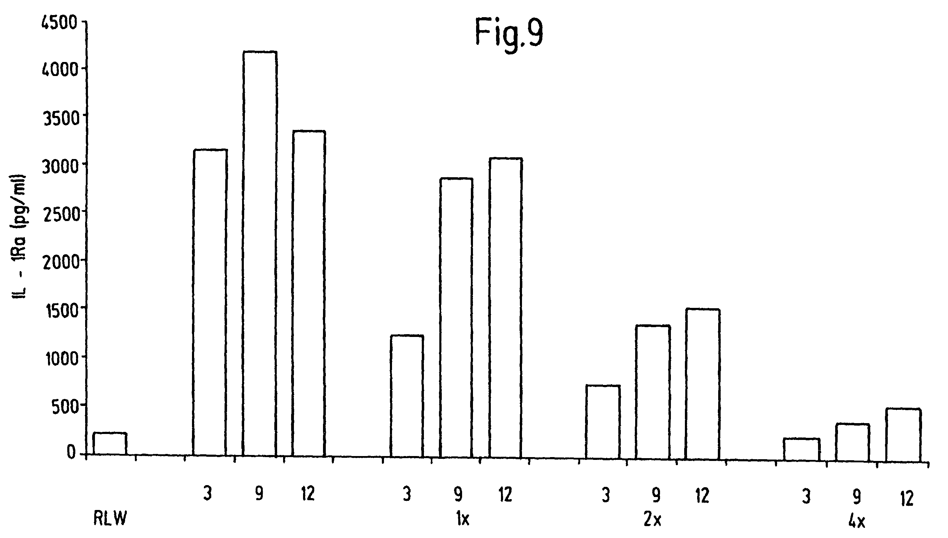

### Fig. 9 IL-1Ra Produktion unter Verwendung einer Mikrotiterplatte mit unterschiedlichen Mengen Glasgranulat und mit verschiedenen Blutkonzentrationen

| Methode | |
|---|---|
| IL-1Ra Produktion | Verwendung einer Mikrotiterplatte wie beschrieben in Beispiel 6. Abweichend jedoch ist nicht 1 ml heparinisiertes Vollblut hinzugegeben, sondern es ist soviel heparinisiertes Vollblut hinzugegeben worden, bis das Endvolumen 1 ml betrug |
| | |
| Glaskugeln | Duran, Chromschwefelsäure behandelt, gewaschen und autoklaviert |
| | |
| 3, 9, 12 | = Hinzufügung von 3, 9 oder 12 Kugeln |
| | |
| 1x, 2x, 4x | = 1x, 2x bzw. 4x mit RPMI 1640 verdünntes heparinisiertes Vollblut |
| | |
| RLW | Reaktionsleerwert, IL-1Ra Konzentration bei Abnahme (vor der IL-1Ra Produktion) in unverdünntem Blut |

### Ergebnis

Bei jeder Blutverdünnung führt die Erhöhung der Glasgranulat-Menge zu einer Erhöhung der IL-1Ra Produktion. Ein Maximum für die IL-1Ra Produktion wird durch das Verhältnis zwischen der Anzahl der Kugeln und Blutmenge, beziehungsweise der Glasoberfläche und Blutzellenzahl bestimmt.

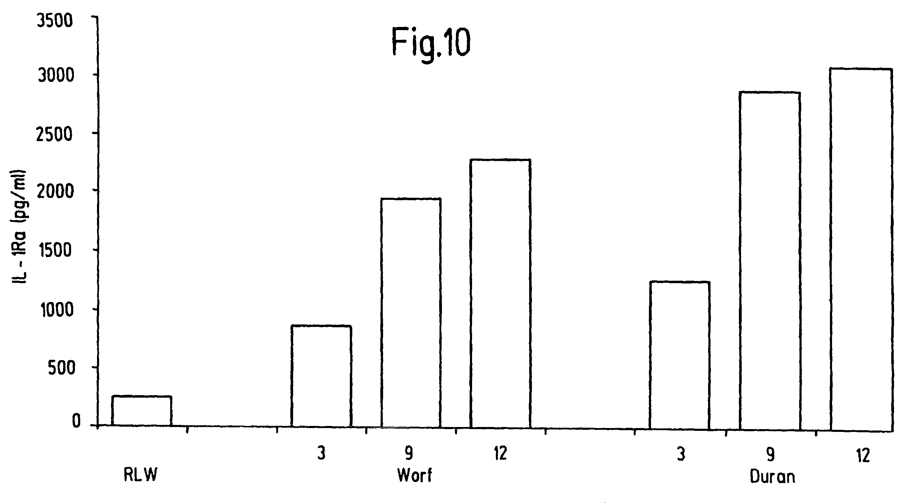

### Fig. 10 IL-1Ra Produktion unter Verwendung einer Mikrotiterplatte mit unterschiedlichen Mengen Glasgranulat und zwei Sorten Glas-Granulat

| Methode | |
|---|---|
| IL-1Ra Produktion | Verwendung einer Mikrotiterplatte wie beschrieben in Beispiel 6 |
| | |
| Glaskugeln | Duran oder Worf, Chromschwefelsäure behandelt, gewaschen und autoklaviert |
| | |
| 3, 9, 12 | = Hinzufügung von 3, 9 oder 12 Kugeln |
| | |
| RLW | Reaktionsleerwert, IL-1Ra Konzentration bei Abnahme (vor der IL-1Ra Produktion) |

### Ergebnis

Sowohl das Erhöhen der Kugelzahl beziehungsweise der Glasoberfläche bei Kalknatronglas (am Beispiel Worf) als auch bei Borosilikatglas (am Beispiel Duran) führt zu einer höheren IL-1Ra Produktion

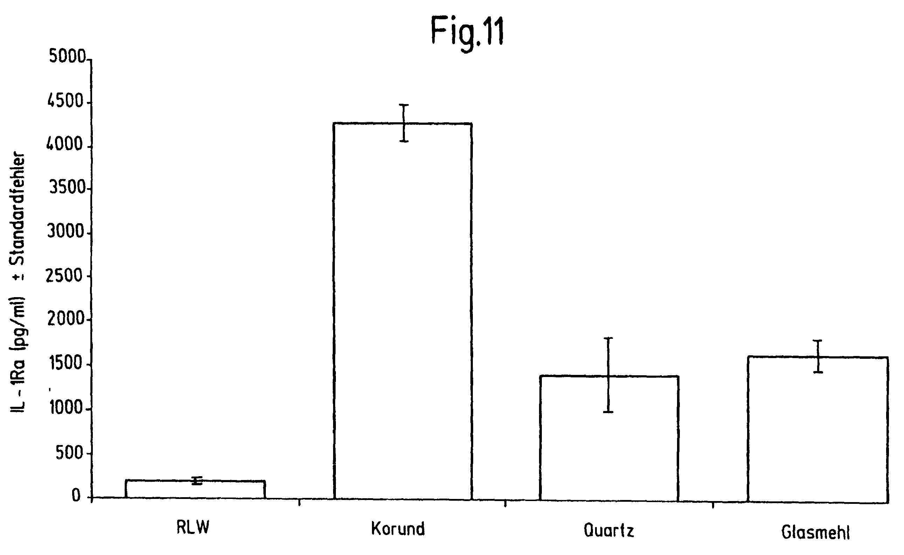

### Fig. 11 IL-1Ra Produktion unter Verwendung einer Mikrotiterplatte mit unterschiedlichen Mengen Glasgranulat und zwei Sorten Glasgranulat

| Methode | |
|---|---|
| IL-1Ra Produktion | Verwendung einer Mikrotiterplatte wie beschrieben in Beispiel 6 |
| | |
| Glaskugeln | Duran, Chromschwefelsäure behandelt, gewaschen und autoklaviert |
| | |
| RLW | Reaktionsleerwert, IL-1Ra Konzentration bei Abnahme (vor der IL-1Ra Produktion) |
| | |
| Korund | alpha Al₂O₃, Suspension |
| | |
| Quarz | Quarzsand, 220 mg/ml |
| | |
| Glasmehl | 0,2 mg/ml |

### Ergebnis

Das Hinzufügen von Silikatoxiden oder Aluminiumoxiden, die Bestandteile der beschriebenen Glasgranulate sind (siehe technische Daten des Glasgranulats), in der Form von Korund beziehungsweise Quarzsand führt zu einer hohen IL-1Ra Produktion

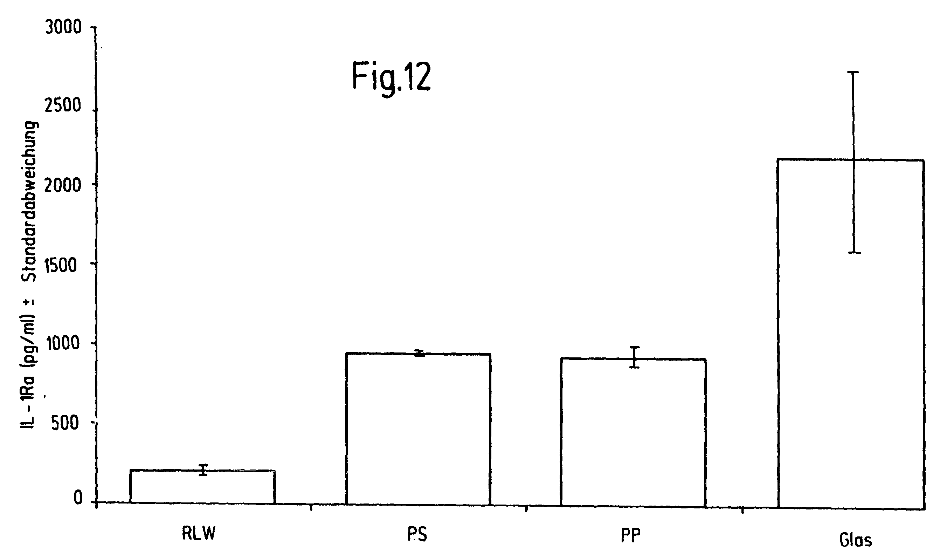

### Fig. 12 IL-1Ra Produktion unter unterschiedlichen Behältermaterialien

| Methode | |
|---|---|
| IL-1Ra Produktion | Verwendung einer Mikrotiterplatte wie beschrieben in Beispiel 6 |
| | |
| Glaskugeln | Duran, Chromschwefelsäure behandelt, gewaschen und autoklaviert |
| | |
| RLW | Reaktionsleerwert, IL-1Ra Konzentration bei Abnahme (vor der IL-1Ra Produktion) |
| | |
| PS | Polystyrol, Mikrotiterplatte (Firma Nunc, Art. Nr. 150687) |
| | |
| pp | Polypropylen, Reaktionsgefäß (Firma Sarsted, Art. Nr. 62/554.502) |
| | |
| Glas | handelsübliches Reaktionsgefäß, autoklaviert |

### Ergebnis

Sowohl in Glas- als auch in Kunststoffbehältern kann IL-1Ra produziert werden, jedoch ist die Effektivität der IL-1Ra Produktion in Glasbehältern signifikant höher

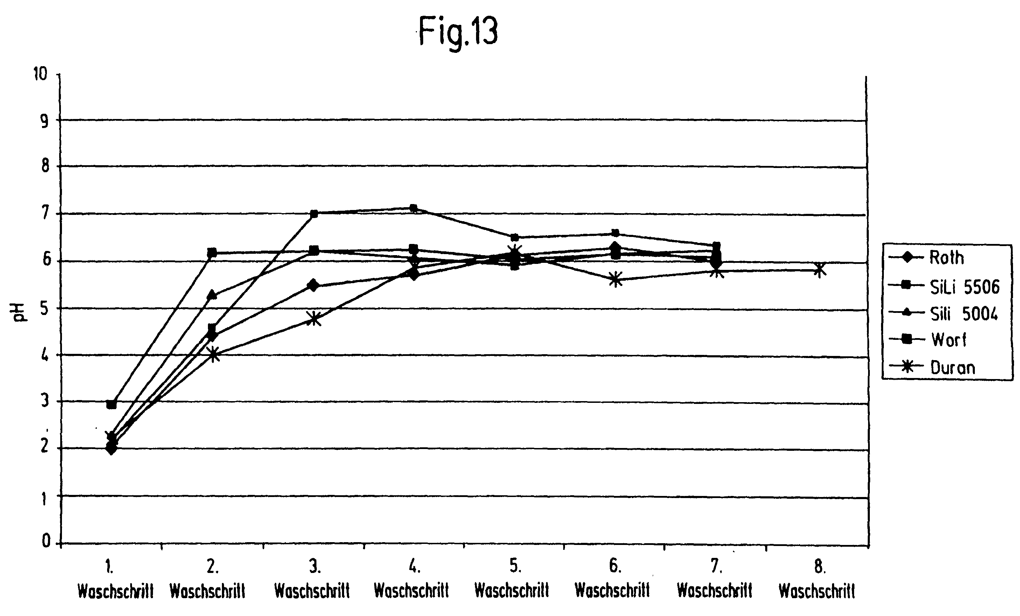

### Fig. 13 pH in dem Waschwasser nach Chromschwefelsäure-Behandlung des Glasgranulats

| Methode | |
|---|---|
| Glaskugeln | alle aufgelisteten Kugeln wurden mit Chromschwefelsäure behandelt, wurde der pH-Wert nach jedem Waschschritt mit Hilfe eines pH-Meters bestimmt |
| | |
| | Gewaschen wurde mit Reinstwasser (Biochrom Reinstwasser, Art. Nr. L 0040), pH zwischen 6,0 und 6,5 |

### Ergebnis

Nach der Chromschwefelsäure-Behandlung konnten für alle beschriebenen Kugeln alle Säurerester weggewaschen werden

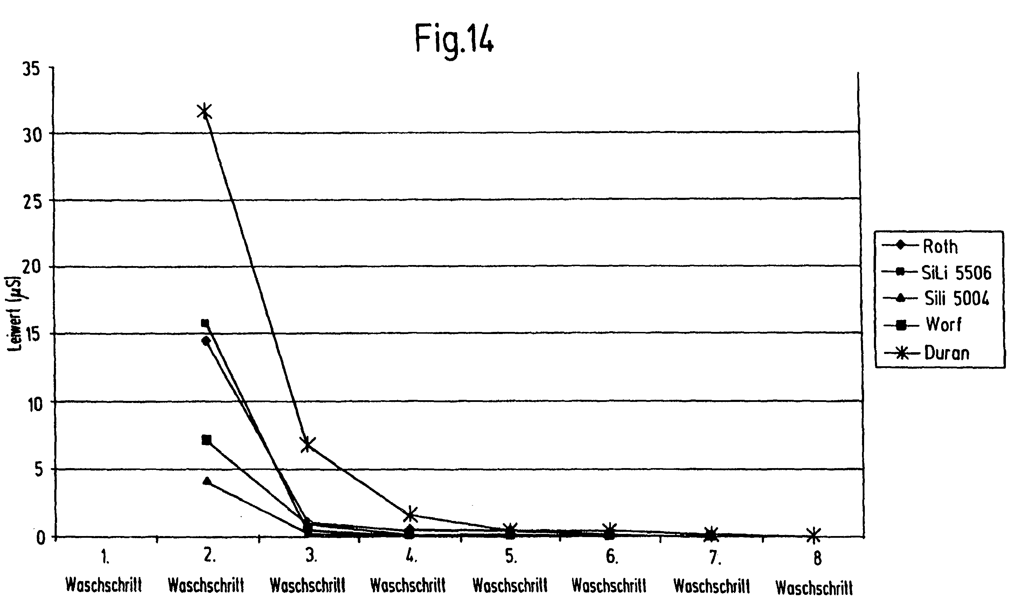

### Fig. 14 Leitwert in dem Waschwasser nach Chromschwefelsäure-Behandlung des Glasgranulats

| Methode | |
|---|---|
| Glaskugeln | alle aufgelisteten Kugeln wurden mit Chromschwefelsäure behandelt, anschließend wurde der Leitwert des Waschwassers nach jedem Waschschritt mit Hilfe eines Leitwertmessers bestimmt |
| | |
| | Gewaschen wurde mit Reinstwasser (Biochrom Reinstwasser, Art. Nr. L 0040), Leitwert 0 µS |

### Ergebnis

Nach Chromschwefelsäure-Behandlung und Waschen konnten für alle beschriebenen Kugeln keine Auswaschungen festgestellt werden. Damit kann die IL-1Ra Induktion durch mögliche pyrogenwirkende Auswaschungen ausgeschlossen werden

### Technische Daten des Glasgranulats

### 1.1 Roth

| | |
|---|---|
| **Größe** | 2,85-3,3 |
| **Material** | Chem. Zus. s.u. |
| **Chem. Zusammensetzung (%)** | |
| SiO2 | 68 |
| CaO | 3 |
| BaO | 6 |
| K2O | 8 |
| Na2O | 10 |
| Al2O3 | 1 |
| B2O3 | 2 |
| Bleifrei | |

### 1.2 SiLi 5506/89-6

| | |
|---|---|
| **Größe** | 2,3-2,5 mm |
| **Material** | Borosilikatglas |
| **Behandlung** | 1. Schleifverfahren |
| | 2. Polierverfahren |
| **Chem. Zusammensetzung (%)** | |
| SiO2 | 82 |
| Na2O | 2 |
| Al2O3 | 2 |
| B2O3 | 14 |
| | |
| **Spezifisches Gewicht (kg/dm3)** | 223 |
| **Härte nach Mohs** | 7 |
| **Linearer Ausdehnungskoeffizient (20-300°C)** | 325 |
| **Hydrolytische Klasse (DIN ISO 719)** | 1 |
| **Säureklasse (DIN 12116)** | 1 |
| **Laugenklasse (DIN ISO 695)** | 2 |
| **Transformationstemperatur (°C)** | 530 |

### 1.3 SiLi 5004/99-5

| | |
|---|---|
| **Größe** | 2,5 mm |
| **Material** | Kalknatronglas |
| **Behandlung** | Pressverfahren |
| **Chem. Zusammensetzung (%)** | |
| SiO2 | 67 |
| Na2O | 16 |
| CaO | 7 |
| Al2O3 | 5 |
| B2O3 | 3 |
| MgO | 2 |
| PbO | frei |
| | |
| **Härte nach Mohs** | >= 6 |

### 1.4 Worf

| | |
|---|---|
| **Größe** | 2 bis zu 3,5 mm |
| **Material** | Kalknatronglas |
| **Behandlung** | poliert und thermisch verfestigt |
| **Chem. Zusammensetzung (%)** | |
| SiO2 | 65 |
| Na2O | 16 |
| CaO | 7 |
| Al2O3 | 5 |
| B2O3 | 3 |
| MgO | 2 |
| Bleigehalt | ohne |
| | |
| **Dichte (g/dm3)** | 2,54 |
| **Härte nach Mohs** | 6 |
| **Hydrolytische Klasse** | 3 |
| **Deformationstemperatur (°C)** | 530 +/- 10 |

### 1.5 Duran

| | |
|---|---|
| **Größe** | 2 bis zu 3,5 mm |
| **Material** | Borosilikatglas |
| **Behandlung** | 1. Schleifverfahren |
| | 2. Polierverfahren |
| **Chem. Zusammensetzung (%)** | |
| SiO2 | 81 |
| Na2O+K2O | 4 |
| Al2O3 | 2 |
| B2O3 | 13 |
| | |
| **Dichte (g/dm3)** | **2,23)** |
| **Linearer Ausdehnungskoeffizient (20-300°C)** | 3,25 |
| **Hydrolytische Klasse (DIN ISO 719)** | 1 |
| **Säureklasse (DIN 12116)** | 1 |
| **Laugenklasse (DIN ISO 695)** | 2 |
| **Transformationstemperatur (°C)** | 525 |

## Patentansprüche

1. Verfahren zur Herstellung von IL-1Ra in einer Spritze, wobei die Spritze mit einer Körperflüssigkeit eines Organismus gefüllt, inkubiert und das IL-1Ra in der Körperflüssigkeit gebildet wird.

2. Verfahren nach Anspruch 1, wobei die Spritze aus Glas, Kunststoff, Korund, und/oder Quarz besteht oder diese(s) enthält.

3. Verfahren nach Anspruch 2, wobei der Kunststoff Polystyrol, Polyvinylchlorid, Polyethylen oder Polypropylen ist.

4. Verfahren nach einem der Ansprüche 1, 2 oder 3, wobei die Spritze eine modifizierte innere Struktur aufweist.

5. Verfahren nach einem der Ansprüche 1 bis 4, wobei die Spritze eine mittels eines ätzenden Agens modifizierte innere Struktur aufweist.

6. Verfahren nach einem der Ansprüche 1 bis 3, wobei die Spritze eine unmodifizierte innere Struktur aufweist.

7. Verfahren nach einem der vorhergehenden Ansprüche, wobei die innere Struktur der Spritze durch ihre innere Oberfläche, insbesondere oberflächenvergrößernde Ausformungen, gebildet wird.

8. Verfahren nach einem der vorhergehenden Ansprüche, wobei die innere Struktur durch in der Spritze vorhandene Perlen, Kugeln, Gele, Wolle, Mehl, Granulate oder Partikel aus Glas, Kunststoff, Korund und/oder Quarz gebildet wird.

9. Verfahren nach einem der Ansprüche 4 bis 8, wobei die innere Struktur aus Polystyrol, Polyvinylchlorid, Polyethylen, Polypropylen besteht oder dieses enthält.

10. Verfahren nach einem der vorhergehenden Ansprüche, wobei das ätzende Agens eine Lauge oder eine Säure, insbesondere Chromschwefelsäure, ist.

11. Verfahren nach einem der vorhergehenden Ansprüche, wobei die Körperflüssigkeit Blut ist.

12. Verfahren zur Herstellung einer zur In-vitro-Induktion von IL-1Ra geeigneten Spritze, wobei die Spritze mit einem ätzenden Agens gefüllt, inkubiert und das Agens entfernt wird.

13. Verfahren nach Anspruch 12, wobei die innere Struktur der Spritze aus Polystyrol, Polypropylen, Polyvinylchlorid, Polyethylen, Korund, Quarz oder Glas besteht oder dieses enthält.

14. Verfahren nach einem der Ansprüche 12 oder 13, wobei das ätzende Agens eine Lauge oder Säure, insbesondere Chromschwefelsäure, ist.

15. Verfahren nach einem der Ansprüche 12 bis 14, wobei nach dem Entfernen des ätzenden Agens die innere Struktur der Spritze gewaschen und gegebenenfalls sterilisiert wird.

16. Spritze, insbesondere hergestellt nach einem der Ansprüche 12 bis 15, **dadurch gekennzeichnet, dass** die innere Struktur der Spritze aus Kunststoff, insbesondere Polystyrol, Polyvinylchlorid, Polyethylen oder Polypropylen, Korund, Quarz oder Glas besteht oder dieses enthält und mittels eines ätzenden Agens zur Erzeugung einer modifizierten Oberfläche der inneren Strukturen behandelt wurde.

## Claims

1. Method of manufacture of IL-1Ra in a syringe, where the syringe is filled with a bodily fluid from an organism, incubated and the IL-1Ra is formed in the bodily fluid.

2. Method according to claim 1, in which the syringe is made of or contains glass, plastic, corundum and/or quartz.

3. Method according to claim 2, where the plastic is polystyrene, polyvinyl chloride, polyethylene or polypropylene.

4. Method according to one of claims 1, 2 or 3, where the syringe displays a modified internal structure.

5. Method according to one of claims 1 to 4, where the syringe displays a modified internal structure by means of an etching agent.

6. Method according to one of claims 1 to 3, where the syringe displays an unmodified internal structure.

7. Method according to one of the preceding claims where the internal structure of the syringe is formed by its internal surface, in particular surface-enlarging projections.

8. Method according to one of the preceding claims where the internal structure is formed by pearls, spheres, gels, wool, flour, granulates or particles made from glass, plastic, corundum and/or quartz present in the syringe.

9. Method according to one of claims 4 to 8, where the internal structure consists of or contains polystyrene, polyvinyl chloride, polyethylene or polypropylene.

10. Method according to one of the preceding claims where the etching agent is an alkali or an acid, in particular chromic-sulphuric acid.

11. Method according to one of the preceding claims where the bodily fluid is blood.

12. Method of manufacture of a syringe suitable for the in vitro induction of IL-1 Ra, where the syringe is filled with an etching agent, incubated and the agent is removed.

13. Method according to claim 12, where the internal structure of the syringe consists of or contains polystyrene, polypropylene, polyvinyl chloride, polyethylene, corundum, quartz or glass.

14. Method according to one of claims 12 or 13, where the etching agent is an alkali or an acid, in particular chromic-sulphuric acid.

15. Method according to one of claims 12 to 14, where after the removal of the etching agent the internal structure of the syringe is washed and sterilised if necessary.

16. Syringe, in particular manufactured according to one of claims 12 to 15, **characterised in that** the internal structure of the syringe consists of or contains plastic, in particular polystyrene, polyvinyl chloride, polyethylene or polypropylene, corundum, quartz or glass and has been treated by means of an etching agent to create a modified surface of the internal structure.

## Revendications

1. Procédé de préparation d'IL-1Ra dans une seringue, dans lequel la seringue est remplie d'un fluide corporel d'un organisme, mise à incuber, puis l'IL-1 Ra est formé dans le fluide corporel.

2. Procédé selon la revendication 1, dans lequel la seringue se compose de verre, de plastique, de corindon et/ou de quartz, ou contient ce(s) matériau(x).

3. Procédé selon la revendication 2, dans lequel le plastique est du polystyrène, du polychlorure de vinyle, du polyéthylène ou du polypropylène.

4. Procédé selon l'une des revendications 1, 2 ou 3, dans lequel la seringue présente une structure interne modifiée.

5. Procédé selon l'une des revendications 1 à 4, dans lequel la seringue présente une structure interne modifiée par un agent caustique.

6. Procédé selon l'une des revendications 1 à 3, dans lequel la seringue présente une structure interne non-modifiée.

7. Procédé selon l'une des revendications précédentes, dans lequel la structure interne de la seringue est formée par sa surface interne, en particulier par des développements augmentant la surface.

8. Procédé selon l'une des revendications précédentes, dans lequel la structure interne est formée par des perles, des billes, des gels, de la laine, de la farine, des granulés ou des particules de verre, de plastique, de corindon et/ou de quartz présents dans la seringue.

9. Procédé selon l'une des revendications 4 à 8, dans lequel la structure interne se compose de polystyrène, de polychlorure de vinyle, de polyéthylène, de polypropylène, ou contient ceux-ci.

10. Procédé selon l'une des revendications précédentes, dans lequel l'agent caustique est une lessive ou un acide, en particulier de l'acide sulfochromique.

11. Procédé selon l'une des revendications précédentes, dans lequel le fluide corporel est du sang.

12. Procédé de préparation d'une seringue appropriée pour l'induction in vitro d'IL-1Ra, dans lequel la seringue est remplie d'un agent caustique, mise à incuber, puis l'agent est retiré.

13. Procédé selon la revendication 12, dans lequel la structure interne de la seringue se compose de polystyrène, de polypropylène, de polychlorure de vinyle, de polyéthylène, de corindon, de quartz ou de verre, ou en contient.

14. Procédé selon l'une des revendications 12 ou 13, dans lequel l'agent caustique est une lessive ou un acide, en particulier de l'acide sulfochromique.

15. Procédé selon l'une des revendications 12 à 14, dans lequel, après enlèvement de l'agent caustique, la structure interne de la seringue est lavée et éventuellement stérilisée.

16. Seringue, en particulier fabriquée selon l'une des revendications 12 à 15, **caractérisée en ce que** la structure interne de la seringue se compose de plastique, en particulier de polystyrène, de polychlorure de vinyle, de polyéthylène ou de polypropylène, de corindon, de quartz ou de verre, ou en contient, et a été traitée au moyen d'un agent caustique pour générer une surface modifiée des structures internes.
